# EUROPEAN PATENT APPLICATION

(11) **EP 1 860 109 A1**
(43) Date of publication of application: **28.11.2007**
(21) Application number: 06114491.1
(22) Date of filing: 24.05.2006
(51) Int. Cl.: C07D 491/04, A61K 31/519, A61P 29/00

(54) **Azetidine amino pyrimidine compounds for the treatment of inflammatory disorders**

(71) Applicant: Cellzome (UK) Ltd., CB10 1XL Cambridgeshire Little Chesterford (GB)
(72) Inventor: Reid, Alison, Cambridgeshire CB4 8SA (GB); Wilson, Francis, Hertfordshire AL7 4QJ (GB); Dyke, Hazel, Harlow, Essex CM19 5TR (GB); Price, Steve, Harlow, Essex CM19 5TR (GB); Cramp, Sue, Harlow, Essex CM19 5TR (GB)
(74) Representative: Dick, Alexander

(57) **Abstract**

The present invention relates to novel azetidine amino pyrimidine compounds of formula (I) for the modulation of the histamine H4 receptor and the treatment or prevention of conditions mediated by the histamine H4 receptor. The invention also relates to the preparation of such compounds.

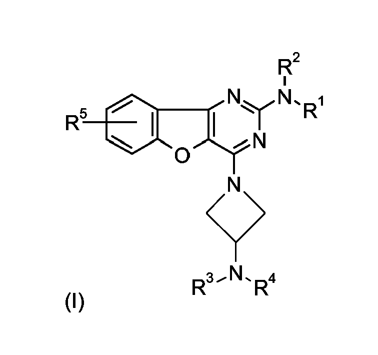

## Description

The present invention relates to novel fused heterocyclic compounds, for the modulation of the histamine H4 receptor and the treatment or prevention of conditions mediated by the histamine H4 receptor. The invention also relates to the preparation of such compounds.

Histamine is a biogenic amine important in the regulation of different physiological processes in the body. Histamine is synthesized, by histidine decarboxylation, from L-histidine in specific cell types such as mast cells, basophils and neurons. Histamine binds to cell membrane receptors of the G-protein coupled receptors family (GPCRs). Three different histamine receptors have been first identified: H1, H2 and H3 (reviewed in S. J. Hill et al., International Union of Pharmacology. XIII. Pharmacol Rev 1997 49: 253-278). H1 receptors trigger smooth muscle contractions and play an important role in allergy. H2 receptors regulate gastric acid secretion in the stomach and H3 receptors control release of histamine and neurotransmitters by neurons. Recently a new histamine receptor (H4) has been identified and it has the highest similarity to the H3 receptor (35% overall amino acid similarity and 58% similarity in the transmembrane region) (Oda T et al., J Biol Chem. 2000 Nov 24; 275(47):36781-6, Zhu Y et al., Mol Pharmacol. 2001 Mar; 59(3):434-41, Morse KL et al., J Pharmacol Exp Ther. 2001 Mar; 296(3):1058-66, Nguyen T et al., Mol Pharmacol. 2001 Mar; 59(3):427-33., Liu C et al., Mol Pharmacol. 2001 Mar; 59(3):420-6, Nakamura T et al., Biochem Biophys Res Commun. 2000 Dec 20; 279(2):615-20).

The H4 receptor is expressed mainly in cells of the hemopoietic lineage, especially mast cells, eosinophils and basophils (Oda T et al., J Biol Chem. 2000 Nov 24; 275(47):36781-6, Zhu Y et al., Mol Pharmacol. 2001 Mar; 59(3):434-41, Morse KL et al., J Pharmacol Exp Ther. 2001 Mar; 296(3):1058-66, Liu C et al., Mol Pharmacol. 2001 Mar; 59(3):420-6). The H4 receptor is also expressed in lower species (mouse, rat, guinea pig) and its tissue distribution is similar to human. However H4 receptor sequence in mouse, rat, and guinea pig is significantly different from the human H4 sequence (69%, 68%, 65% similarity respectively) and this translates in differences in binding affinities to histamine and H3/H4 ligands, as well as differences in signal transduction response (Liu C et al., J Pharmacol Exp Ther. 2001 Oct; 299(1):121-30).

H4 receptors have been shown to mediate chemotaxis and calcium mobilization in mast cells (Hofstra CL et al., J Pharmacol Exp Ther. 2003 Jun; 305(3):1212-21) and eosinophil chemotaxis with cell shape change and up regulation of adhesion molecules (Ling P et al., Br J Pharmacol. 2004 May; 142(1):161-71. Erratum in: Br J Pharmacol. 2004 Jul; 142(6):1052, Buckland KF et al., Br J Pharmacol. 2003 Nov; 140(6):1117-27, O'Reilly M et al., J Recept Signal Transduct Res. 2002 Feb-Nov; 22(1-4):431-48). H4 is also implicated in histamine-induced interleukin-16 release from human CD8+ T cells (Gantner F et al., J Pharmacol Exp Ther. 2002 Oct; 303(1):300-7), in leukotriene B4 production and mast cell dependent neutrophil recruitment (Takeshita K et al., J Pharmacol Exp Ther. 2003 Dec; 307(3):1072-8). All these data indicate that the histamine H4 receptor may play a role in the inflammatory response.

An indole amide, JNJ7777120, has been recently described as a potent and selective Histamine H4 receptor antagonist, (Ki 4.5 nM. pA2 8.1. Equipotent against human, mouse and rat receptors. >1000-fold selectivity over H1, H2, or H3 receptor and no cross binding against 50 other targets) (Thurmond RL et al., J Pharmacol Exp Ther. 2004 Apr; 309(1):404-13). JNJ7777120, *in vitro,* blocks histamine-induced chemotaxis and calcium influx in mouse mast cells, and *in vivo* histamine induced migration of tracheal mast cells from the connective tissue to the epithelium in mice. JNJ7777120 reduces infiltration in a mouse zymosan-induced peritonitis model indicating a role of the histamine H4 receptor in an inflammatory process *in vivo.*

Therefore, it is believed that modulators of the histamine H4 receptor have utility in a variety of inflammation disorders.

Background material about inflammation can be found in the following reviews: Tracey KJ., Nature. 2002 Dec 19-26; 420(6917):853-9, Nathan C., Nature. 2002 Dec 19-26; 420(6917):846-52.

Inflammation herein refers to the response that develops as a consequence of histamine release that can be caused by immunological and non-immunological stimuli. Inflammation can be due to any one or a plurality of conditions including, but not limited to, allergy, allergic rhinitis and asthma. In terms of the development of the disorder the inflammatory conditions include, but are not limited to, acute inflammation, allergic inflammation and chronic inflammation.

There is a need in the art for new effective modulators of the histamine H4 receptor.

Thus, the object of the present invention is to provide a new class of histamine H4 receptor modulators which may be effective, preferably in the treatment of inflammatory diseases.

Accordingly, the present invention provides compounds of the formula (I) 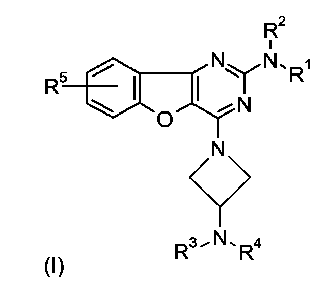 or a pharmaceutically acceptable salt, ester, prodrug or metabolite thereof, wherein
R¹ and R² are independently selected from the group consisting of H, -C(O)CH₃, -S(O)₂CH₃, CH₃, CH₂CH₃, CH₂F, CHF₂, CF₃, and cyclopropyl;
R³ and R⁴ are independently selected from the group consisting of H, CH₃, CH₂CH₃, CH₂F, CHF₂, CF₃, and cyclopropyl;
R⁵ is H, F, Cl, Br, CH₃, CN, CF₃, OH, OCH₃, or OCF₃.

Preferred compounds of formula (I) are those compounds in which one or more of the residues contained therein have the meanings given below, with all combinations of preferred substituent definitions being a subject of the present invention.

Preferably, R¹, R² are independently selected from the group consisting of H, CH₃ and CH₂CH₃.

Preferably, R¹ and R² are H, R¹ and R² are CH₃ or when R¹ and R² are different one of them is H.

Preferably, R³, R⁴ are independently selected from the group consisting of H, CH₃, and CH₂CH₃.

Preferably, R³ and R⁴ are H, or when R³ and R⁴ are different one of them is H.

Preferably, R⁵ is in 8-position.

Preferably, R⁵ is Cl.

Preferred compounds are those given below:
[1-(2-Ethylamino-8-chlorobenzo[4,5]furo[3,2-d]-pyrimidin-4-yl)azetidin-3-yl] N-ethyl amine;
N-[1-(2-Amino-8-chlorobenzo[4,5]furo[3,2-d]pyrimidin-4-yl)azetidin-3-yl] N-methyl amine.

Some of the compounds of the inventions and/or salts or esters thereof, will exist in different stereoisomeric forms. All of these forms are subjects of the invention.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) may be obtained from stereoselective synthesis using optically pure starting materials.

Furthermore, the invention relates to any of the compounds according to the invention and/or a pharmaceutically acceptable salt or ester thereof, especially for use as a medicament.

Described below are exemplary salts of the compounds according to the invention which are included herein. The list of the different salts stated below is not meant to be complete and limiting.

Compounds according to the invention which contain one or more basic groups, i.e. groups which can be protonated can be used according to the invention in the form of their addition salts with inorganic or organic acids.

Examples for suitable salts include hydrogen chloride, hydrogen bromide, phosphoric acid, sulfuric acid, nitric acid, methanesulfonic acid, p-toluenesulfonic acid, napthalenedisulfonic acid, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid and other acids known to a person skilled in the art.

The term "pharmaceutically acceptable" means approved by a regulatory agency such as the EMEA (Europe) and/or the FDA (US) and/or any other national regulatory agency for use in animals, preferably in humans.

The respective salts of the compounds according to the invention can be obtained by customary methods which are known to the person skilled in the art, for example by contacting these with an organic or inorganic acid or base in a solvent or dispersant, or by anion exchange or cation exchange with other salts.

Furthermore, the invention includes all salts of the compounds according to the invention which, owing to low physiological compatibility, are not directly suitable for use in pharmaceuticals but which can be used, for example, as intermediates for chemical reactions or for the preparation of pharmaceutically acceptable salts or which might be suitable for the H4 antagonist activity of a compound according of the invention in any suitable manner, such as any suitable in vitro assay.

The present invention furthermore includes all solvates of the compounds according to the invention.

The present invention furthermore includes derivatives/prodrugs (including the salts thereof) of the compounds according to the invention which contain physiologically tolerable and cleavable groups and which are metabolized in animals, preferably mammals, most preferably humans into a compound according to the invention.

The term "prodrug" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of a prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

The present invention furthermore includes the metabolites of the compounds according to the invention.

The term "metabolites" refers to all molecules derived from any of the compounds according to the invention in a cell or organism, preferably mammal.

Preferably the term relates to molecules which differ from any molecule which is present in any such cell or organism under physiological conditions.

The structure of the metabolites of the compounds according to the invention will be obvious to any person skilled in the art, using the various appropriate methods

Another object of the invention is a pharmaceutical composition comprising a compound according to the present invention in a mixture with an inert carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by mixing a compound of the present invention and a pharmaceutically acceptable carrier.

Any suitable route of administration may be employed for providing a mammal, especially a human, with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The pharmaceutical compositions of the present invention comprise a compound of formula (I) as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

The compositions include compositions suitable for oral, rectal, topical, parenteral (including subcutaneous, intramuscular, and intravenous), ocular (ophthalmic), pulmonary (aerosol inhalation), or nasal administration, although the most suitable route in any case will depend on the nature and severity of the conditions being treated and on the nature of the active ingredient. They may be conveniently presented in unit dosage form and prepared by any of the methods well-known in the art of pharmacy.

For administration by inhalation, the compounds of the present invention are conveniently delivered in the form of an aerosol spray presentation from pressurized packs or nebulizers. The compounds may also be delivered as powders which may be formulated and the power composition may be inhaled with the aid of insufflation powder inhaler device. The preferred delivery systems for inhalation are metered dose inhalation (MDI) aerosol, which may be formulated as a suspension or solution of a compound of formula (I) in suitable propellants, such as fluorocarbons or hydrocarbons and dry powder inhalation (DPI) aerosol, which can be formulated as a dry powder of a compound of formula (I) with or without additional excipients.

Suitable topical formulations of a compound of formula (I) include transdermal devices, aerosols, creams, ointments, lotions, dusting powders and the like.

In practical use, the compounds of formula (I) can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous). In preparing the compositions for oral dosage form, any of the usual pharmaceutical media may be employed, such, as, for example, water, glycols, oils, alcohols, flavouring agents, preservatives, colouring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

In addition to the common dosage forms set out above, the compounds of formula (I) may also be administered by controlled release means and/or delivery devices such as those described in U.S. patents 3845770, 3916899, 3536809, 3598123, 3630200 and 4008719.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Moulded tablets may be made by moulding in a suitable machine, a mixture of the powdered compound moistened with an inert liquid diluent. Desirably, each tablet contains from about 1 mg to about 500 mg of the active ingredient and each cachet or capsule contains from about 1 to about 500 mg of the active ingredient.

The following are examples of representative pharmaceutical dosage forms for the compounds of formula (I):

### Injectable Suspension (I.M.):

| | |
|---|---|
| Compound of formula (I) | 10 mg / mL |
| Methylcellulose | 5,0 mg / mL |
| Tween 80 | 0,5 mg / mL |
| Benzyl alcohol | 9,0 mg / mL |
| Benzalkonium chloride | 1,0 mg / mL |

Plus water for injection to a total volume of 1mL

### 500 mg Tablet:

| | |
|---|---|
| Compound of formula (I) | 25 mg / tablet |
| Microcrystalline Cellulose | 415 mg/mL |
| Povidone | 14,0 mg/mL |
| Pregelatinized Starch | 43,5 mg/mL |
| Magnesium Stearate | 2,5 mg/mL |

### 600 mg Capsule:

| | |
|---|---|
| Compound of formula (1) | 25 mg / tablet |
| Lactose Powder | 573,5 mg / tablet |
| Magnesium Stearate | 1,5 mg / tablet |

### Aerosol:

| | |
|---|---|
| Compound of formula (I) | 24 mg / canister |
| Lecithin, NF Liq. Conc. | 1,2 mg / canister |
| Trichlorofluoromethane, NF | 4,025 g / canister |
| Dichlorodifluoromethane, NF | 12,15 g / canister |

Compounds of formula (I) may be used in combination with other drugs that are used in the treatment/prevention/suppression or amelioration of the diseases or conditions for which compounds of formula (I) are useful. Such other drugs may be administered, by a route and in an amount commonly used therefore, contemporaneously or sequentially with a compound of formula (I). When a compound of formula (I) is used contemporaneously with one or more other drugs, a pharmaceutical composition containing such other drugs in addition to the compound of formula (I) is preferred. Accordingly, the pharmaceutical compositions of the present invention include those that also contain one or more other active ingredients, in addition to a compound of formula (I).

Another object of the present invention is a compound according to the invention for use as a medicament.

Yet another object of the present invention is the use of a compound of the present invention for the manufacture of a medicament for the treatment or prophylaxes of one or more diseases or disorders associated with the modulation of histamine H4 receptor, especially with the inflammatory response mediated by histamine H4 receptor.

Yet another object of the present invention is the use of a compound according to the present invention for the manufacture of a medicament for the treatment or prophylaxes of inflammatory diseases.

Yet another object of the present invention is the use of a compound according to the present invention for the manufacture of a medicament for the treatment or prophylaxes of one or more diseases or disorders selected from the group consisting of asthma, psoriasis, rheumatoid arthritis, Crohn's disease, inflammatory bowel disease, ulcerative colitis, allergic rhinitis and other allergic diseases, atopic dermatitis and other dermatological disorders.

Yet another object of the present invention is a method for treating, controlling, delaying or preventing in a mammal, preferably a human patient, in need of treatment of one or more conditions associated with the modulation of histamine H4 receptor, wherein the method comprises the administration of said patient of a pharmaceutically effective amount of a compound according to the present invention.

The present invention is also concerned with processes for preparing the compounds of this invention.

The compounds of formula (I) of the present invention can be prepared according to the procedures of the following schemes and examples, using appropriate materials, and are further exemplified by the following specific examples. Moreover, by utilising the procedures described with the disclosure contained herein, one of ordinary skill in the art can readily prepare additional compounds of the present invention claimed herein. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The compounds of the invention of formula (I) may be isolated in the form of their pharmaceutically acceptable salts, such as those described previously herein above. The free acid form corresponding to isolated salts can be generated by neutralisation with a suitable acid such as acetic acid and hydrochloric acid and extraction of the liberated free acid into an organic solvent followed by evaporation. The free acid form isolated in this manner can be further converted into another pharmaceutically acceptable salt by dissolution in an organic solvent followed by addition of the appropriate base and subsequent evaporation, precipitation, or crystallisation.

It may be necessary to protect reactive functional groups (e.g. hydroxy, amino, thio or carboxy) in intermediates used in the preparation of compounds of formula (I) to avoid their unwanted participation in a reaction leading to the formation of compounds of formula (I). Conventional protecting groups, for example those described by T. W. Greene and P. G. M. Wuts in "Protective groups in organic chemistry" John Wiley and Sons, 1999, may be used.

Compounds of the invention of formula (I) may conveniently be prepared by the reaction in an inert solvent, usually under elevated temperatures, of a cyclic amine of formula (IV) and a compound of formula (V) in which R⁸ represents a suitable leaving group; suitable leaving groups at R⁸ include chloro, bromo, alkylsulphinyl, and alkylsulphonyl. Alternatively the reaction of intermediate (V), in which R⁸ is a halo group such as chloro or bromo, with an cyclic amine intermediate of formula (IV) may be achieved in the presence of a palladium catalyst such as a mixture of palladium bis(trifluoroacetate) and tri(*tert*-butyl)phosphine.

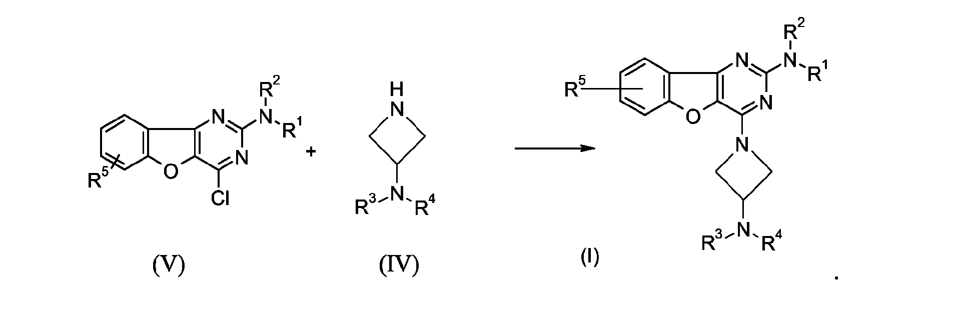

It will be understood by those practiced in the art that the transformation of intermediate (V) to compound (I) by reaction with cyclic amine (IV) when R³ is H may require the presence of a suitable protecting group, for example benzyl, benzoyl or *tert-*butyloxycarbonyl, as may prove most convenient. It is to be understood that if the reaction is carried out on a protected form of cyclic amine (IV) an appropriate deprotection step will be required to obtain the desired compound (I) of the invention in which R³ is H.

Intermediate compounds of formula (V) where R⁸ is chloro or bromo may be prepared, for example, by the Copper catalysed cyclisation of a compound of formula (VI) with a suitable base, for example caesium carbonate or potassium carbonate.

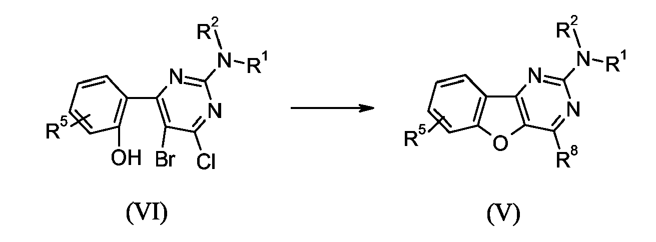

Intermediate compounds of formula (VI) may be prepared, for example, from compounds of formula (VII), by reaction with a suitable Lewis acid. A suitable Lewis acid includes boron trichloride.

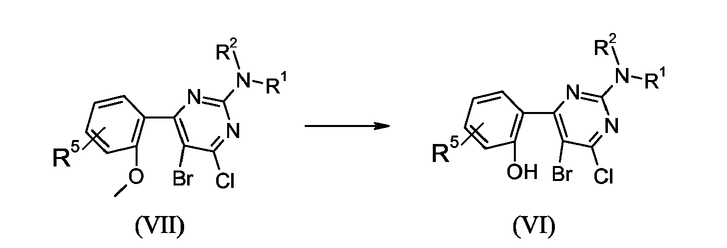

Intermediate compounds of formula (VII) may be prepared from an appropriately substituted dichloropyrimidine of formula (VIII) by reaction with a suitable boronic acid followed by bromination with N-bromosuccinimide.

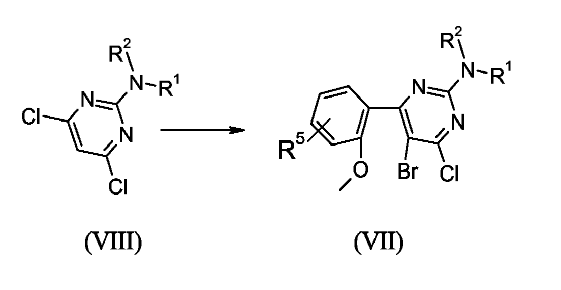

This route can serve as a basis for the preparation of compounds according to the invention by a person skilled in the art.

Another object of the present invention is a process for the preparation of a medicament comprising the steps of:
a) preparing a compound according to a process as outlined above; and
b) formulation of a medicament containing said compound.

The compounds of formula (I) are claimed as having activity as pharmaceuticals, in particular as modulators of histamine H4 receptor, and may be used in the treatment (therapeutic or prophylactic) of conditions/diseases in human and non-human animals, that are known to be at least partially mediated by the activation of the H4 receptor. These compounds could be beneficial for the treatment of inflammatory diseases including, but not limited to asthma, psoriasis, rheumatoid arthritis, Crohn's disease, inflammatory bowel disease, ulcerative colitis, allergic rhinitis and other allergic diseases, atopic dermatitis and other dermatological disorders.

Compounds of the invention of formula (I) can be tested using the following biological test methods to determine their ability to displace histamine from the H4 receptor and for their ability to antagonize the functional effects of histamine at the H4 receptor in a whole cell system.

Radioligand binding assay using histamine H4 receptor transfected CHO K1 membranes.

The receptor binding assay is performed in a final volume of 150 µL binding buffer (50 mM Tris (pH 7.4), 5 mM MgCl₂) using 18nM [2,5-3H]-histamine dihydrochloride (Amersham Biosciences UK Ltd) as the radioligand. Ligands are added in assay buffer containing a constant volume of DMSO (1% v/v). Total binding is determined using 1% v/v of DMSO in assay buffer and non-specific binding is determined using 100 µM of unlabeled histamine dihydrochloride (Sigma). The reaction is initiated with 20 µg of human histamine H4 receptor containing membranes (Euroscreen, Belgium) and the mixture incubated for 90 minutes at 25°C. The reaction is terminated by rapid filtration through GFB filters pre-blocked with PEI (1 % v/v) using a Packard Cell harvester and the filter washed with 2 x 500 µL / well of cold wash buffer (50 mM Tris (pH 7.4), 5 mM MgCl₂, 0.5 M NaCI). The residual radioligand bound to the filter was determined using a Topcount liquid scintillation counter (Perkin Elmer). Compound IC₅₀ values can be determined using an 8-point dose response curve in duplicate with a semi-log compound dilution series. IC₅₀ calculations may be performed using Excel and XL fit (Microsoft) and this value can be used to determine a Kᵢ value for the test compound using the Cheng-Prusoff equation.

Functional assay using histamine H4 receptor transfected CHO K1 membranes.

The GTPγS binding assay is used as a measure of the functional activation of the histamine H4 receptor using membranes prepared from CHO K1 cells stably transfected with the cDNA for the human histamine H4 receptor (Euroscreen, Belgium). The assay is performed in a 96 well Isoplate (Perkin Elmer) in a final volume of 200 µL assay buffer (20 mM HEPES (pH 7.4), 100 mM NaCl, 10 mM MgCl₂, 10 µg/ml saponin and 10 µM GDP) using 0.1nM GTPγ [35S] (Amersham Biosciences UK Ltd) to measure functional incorporation, and in the case of antagonist studies 150 nM histamine dihydrochloride (EC80 for histamine dihydrochloride) to determine maximal incorporation of GTPγ [35S]. Compounds are added in assay buffer containing a constant volume of DMSO (1 % v/v). Total incorporation is determined in the presence of 1 % v/v of DMSO in assay buffer and non-specific binding is determined using 10 µM of unlabeled GTPγS (Sigma). The incorporation is initiated with 15 µg histamine H4 receptor membranes (Euroscreen, Belgium) and the mixture incubated for 5 minutes at 30 °C. Wheat-Germ agglutinin-coated SPA beads (0.75 mg, Amersham Biosciences UK Ltd) are added and the mixture and incubated for 30 minutes at 30 °C. The plate is centrifuged at 1000 x g for 10 minutes at 30 °C and radioactive incorporation counted in a MicroBeta Counter (Wallac).

### Examples

The invention will now be described in detail with reference to the following examples. It will be appreciated that the invention is described by way of example only and modification of detail may be made without departing from the scope of the invention.

¹H NMR spectra were recorded at ambient temperature using either a Varian Unity Inova (400MHz) spectrometer or a Bruker Advance DRX (400MHz) spectrometer, both with a triple resonance 5mm probe. Chemical shifts are expressed in ppm relative to tetramethylsilane. The following abbreviations have been used: br = broad signal, s = singlet, d = doublet, dd = double doublet, t = triplet, q = quartet, m = multiplet.

High Pressure Liquid Chromatography - Mass Spectrometry (LCMS) experiments to determine retention times and associated mass ions were performed using the following methods:
Method A: Experiments performed on a Micromass Platform LCT spectrometer with positive ion electrospray and single wavelength UV 254nm detection using a Higgins Clipeus C18 5µm 100 x 3.0mm column and a 1 mL / minute flow rate. The initial solvent system was 95% water containing 0.1% formic acid (solvent A) and 5% acetonitrile containing 0.1% formic acid (solvent B) for the first minute followed by a gradient up to 5% solvent A and 95% solvent B over the next 14 minutes. The final solvent system was held constant for a further 5 minutes.
Method B: Experiments performed on a Micromass Platform LC spectrometer with positive and negative ion electrospray and ELS/Diode array detection using a Phenomenex Luna C18(2) 30 x 4.6mm column and a 2 ml / minute flow rate. The solvent system was 95% solvent A and 5% solvent B for the first 0.50 minutes followed by a gradient up to 5% solvent A and 95% solvent B over the next 4 minutes. The final solvent system was held constant for a further 1 minute.

Microwave experiments were carried out using either a Personal Chemistry Smith Synthesizer^{™} or Emrys Optimizer^{™} which use a single-mode resonator and dynamic field tuning, both of which give reproducibility and control. Temperatures from 40-250°C can be achieved, and pressures of up to 20bar can be reached.

### Example 1: Preparation of N-[1-(2-amino-8-chlorobenzo[4,5]furo[3,2-d]pyrimidin-4-yl)azetidin-3-yl] N-methyl amine

### Preparation of 2-Amino-4-chloro-6-(5-chloro-2-methoxyphenyl)pyrimidine

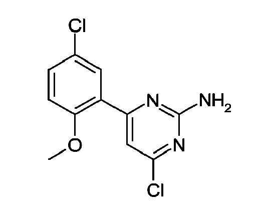

A mixture of 5-chloro-2-methoxyphenylboronic acid (0.744g), 2-amino-4,6-dichloropyrimidine (0.656g), tetrakis-(triphenylphosphine)palladium (0.23g) and cesium carbonate (2.6g) in dimethoxy ethane (16mL) and water (4mL) was irradiated in the microwave at 120°C for 10 minutes. The mixture was diluted with ether and washed with water, saturated aqueous sodium bicarbonate solution and brine then dried (Na₂SO₄ and filtered. The filtrate was evaporated to dryness and the residue was purified by chromatography on silica eluting with a mixture of ether and pentane (1:4 increasing to 3:2) to give 2-amino-4-chloro-6-(5-chloro-2-methoxyphenyl)-pyrimidine (0.757g) as a white powder.

¹H NMR (CDCl₃): δ 3.9 (s, 3H), 5.2 (br s, 2H), 6.95 (d, 1H), 7.3 (s, 1H), 7.35 (dd, 1H), 7.9 (d, 1H).

**Preparation of** 2-Amino-5-bromo-4-chloro-6-(5-chloro-2-methoxyphenyl)-pyrimidine

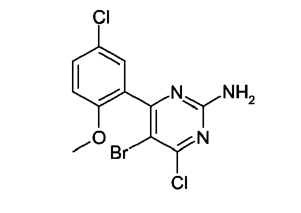

A mixture of 2-amino-4-chloro-6-(5-chloro-2-methoxyphenyl)pyrimidine (0.75g) and N-bromosuccinimide (1.0g) in acetonitrile (30mL) was stirred and heated at reflux for 1.5 hours. The mixture was evaporated to dryness and the residue was dissolved in ether and washed with water, saturated aqueous sodium bicarbonate solution, 10% aqueous sodium metabisulphite solution and brine then dried (Na₂SO₄ and filtered. The filtrate was evaporated to dryness and the residue was purified by chromatography on silica eluting with a mixture of ether and pentane (1:4 increasing to 2:3) to give 2-amino-5-bromo-4-chloro-6-(5-chloro-2-methoxyphenyl)pyrimidine (0.696g) as a tan coloured solid.

¹H NMR (CDCl₃): δ 3.8 (s, 3H), 5.3 (br s, 2H), 6.9 (d, 1H), 7.2 (d. 1H), 7.4 (dd, 1H).

### Preparation of 2-Amino-5-bromo-4-chloro-6-(5-chloro-2-hydroxyphenyl)-pyrimidine

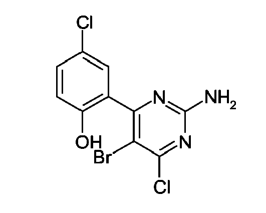

A solution of boron trichloride in dichloromethane (1M, 8mL) was added dropwise to a stirred solution of 2-amino-5-bromo-4-chloro-6-(5-chloro-2-methoxyphenyl)-pyrimidine (0.696g) in dichloromethane (25mL) under an atmosphere of argon. The resultant mixture was stirred and heated at reflux for 2 hours. Further boron trichloride solution in dichloromethane (1M, 2mL) was added and the mixture was stirred and heated at reflux overnight. The mixture was allowed to cool to room temperature and a mixture of ice and water was added. The resultant slurry was partially evaporated and then extracted with ether. The organic phase was washed with saturated aqueous sodium bicarbonate and brine then dried (Na₂SO₄ and filtered. The filtrate was evaporated to dryness to give 2-amino-5-bromo-4-chloro-6-(5-chloro-2-hydroxyphenyl)pyrimidine (0.657g) as a yellow solid.

¹H NMR (CD₃CN): δ 5.9 (br s, 2H), 6.95 (d, 1H), 7.35 (dd, 1H), 7.45 (d, 1H), 8.4 (br s, 1 H).

### Preparation of 2-Amino-4,8-dichlorobenzo[4,5]furo[3,2-d]pyrimidine

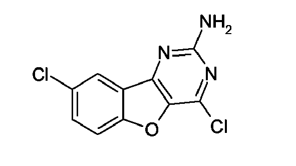

A mixture of 2-amino-5-bromo-4-chloro-6-(5-chloro-2-hydroxyphenyl)pyrimidine (0.388g), copper (I) chloride (0.058g), 2,2,6,6-tetramethylheptan-3,5-dione (0.024mL) and cesium carbonate (0.757g) in toluene (40mL) was stirred and heated at reflux for 24 hours. The resultant mixture was evaporated to dryness and the residue was purified by chromatography on silica eluting with a mixture of methanol and dichloromethane (1:49 gradually increasing to 1:9) to give 2-amino-4,8-dichlorobenzo[4,5]furo[3,2-d]pyrimidine (0.25g) as a beige solid.

¹H NMR (DMSO-d₆): 8 7.2 (s, 2H), 7.75 (dd, 1H), 7.85 (d, 1H), 8.0 (d, 1H).

### Preparation of N-(1-Benzhydrylazetidin-3-yl) N-methyl amine

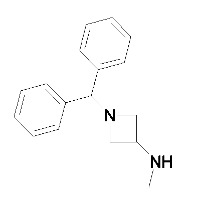

A mixture of 1-benzhydryl-3-methanesulphonyloxyazetidine (1.0g) and a solution of methylamine in ethanol (33%, 10mL) was stirred and heated at reflux for 24 hours. The mixture was cooled to room temperature and evaporated to dryness to give N-(I-benzhydrylazetidin-3-yl) N-methyl amine (1.06g) as a yellow gum.

¹H NMR (CDCl₃) δ 2.45 (s, 3H), 2.95 (m, 2H), 3.4 (br, 1H), 3.5 (m, 3H), 4.4 (s, 1H), 7.2 (m, 2H), 7.25 (m, 4H), 7.4 (m, 4H).

### Preparation of tert-Butyl N-(1-benzhydrylazetidin-3-yl) N-methyl carbamate

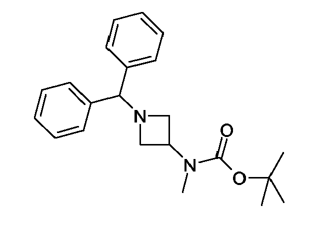

A solution of N-(1-benzhydrylazetidin-3-yl) N-methyl amine (1.06g) in dichloromethane (25mL) was treated with N,N-di*-iso-*propyl N-ethyl amine (1.1mL) and di*-tert-*butyl dicarbonate (0.825g) and the resultant mixture was stirred at room temperature overnight. The mixture was evaporated to dryness and the residue was purified by chromatography on silica eluting with dichloromethane followed by a mixture of ethyl acetate and dichloromethane (1:20) to give *tert*-butyl N-(1-benzhydrylazetidin-3-yl) N-methyl carbamate (0.86g) as a colourless oil.

¹H NMR (CDCl₃) δ 1.4 (s, 9H), 2.9 (s, 3H), 3.0 (m, 2H), 3.45 (t, 2H), 4.35 (s, 1H), 4.5 (br, 1H), 7.2 (m, 2H), 7.25 (m, 4H), 7.4 (m, 4H).

### Preparation of tert-Butyl N-(azetidin-3-yl) N-methyl carbamate

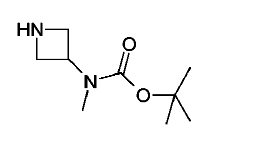

A mixture of *tert-*butyl N-(1-benzhydrylazetidin-3-yl) N-methyl carbamate (0.85g) and palladium hydroxide on carbon (0.128g) in ethanol (20mL) was stirred under an atmosphere of hydrogen for 1 hour. The mixture was filtered through hyflo and the filtrate was evaporated to dryness. The residue was dissolved in methanol and loaded onto an Isolute® SCX-2 column eluting with methanol to remove the unwanted by-products followed by a solution of ammonia in methanol (2M) to give *tert*-butyl N-(azetidin-3-yl) N-methyl carbamate (0.342g) as a colourless oil.

¹H NMR (CDCl₃) δ 1.45 (s, 9H), 2.9 (s, 3H), 3.65 (br, 2H), 3.75 (m, 2H), 4.6-5.0 (br, 1H).

### Preparation of tert-Butyl N-[1-(2-amino-8-chlorobenzo[4,5]furo[3,2-d]pyrimidin-4-yl)azetidin-3-yl] N-methyl carbamate

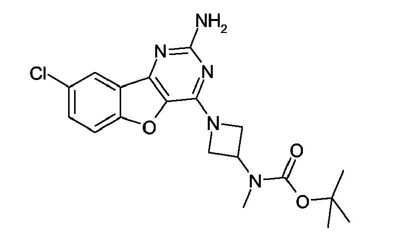

A mixture of 2-amino-4,8-dichlorobenzo[4,5]furo[3,2-d]pyrimidine (0.46g), *tert*-butyl N-(azetidin-3-yl) N-methyl carbamate (0.34g) and N,N-di-*iso*-propyl N-ethyl amine (0.637mL) in ethanol (5mL) was irradiated in the microwave at 120°C for 10 minutes. The mixture was diluted with ethyl acetate and washed with water and brine solution, dried (MgSO₄) and filtered. The filtrate was evaporated to dryness and the residue was purified by chromatography on silica eluting with a mixture of ethyl acetate and dichloromethane (1:1 increasing to neat ethyl acetate) to give *tert*-butyl N-[1-(2-amino-8-chlorobenzo[4,5]furo[3,2-d]pyrimidin-4-yl)azetidin-3-yl] N-methyl carbamate (0.617g) as a yellow solid.

¹H NMR (DMSO-d₆) δ 1.4 (s, 9H), 2.9 (s, 3H), 4.35 (m, 2H), 4.5 (m, 2H), 4.8-5.2 (br, 1H), 6.2 (s, 2H), 7.6 (dd, 1H), 7.7 (d, 1H), 7.85 (d, 1H).

**Product:** N-[1-(2-Amino-8-chlorobenzo[4,5]furo[3,2-d]pyrimidin-4-yl)azetidin-3-yl] N-methyl amine

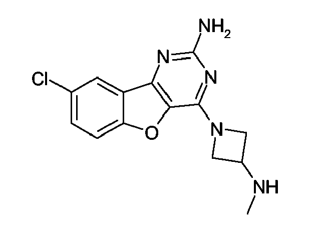

A solution of *tert*-butyl N-[1-(2-amino-8-chlorobenzo[4,5]furo[3,2-d]pyrimidin-4-yl)azetidin-3-yl] N-methyl carbamate (0.61g) in dichloromethane (20mL) was cooled to 0°C and treated with trifluoroacetic acid (10mL) and the resultant solution was stirred at room temperature for 1 hour. The mixture was evaporated to dryness and the residue was redissolved in dichloromethane and washed with saturated aqueous sodium bicarbonate solution then dried (MgSO₄) and filtered. The filtrate was evaporated to dryness and the residue was recrystallised from ethanol to give N-[1-(2-amino-8-chlorobenzo[4,5]furo[3,2-d]pyrimidin-4-yl)azetidin-3-yl] N-methyl amine (0.161g) as an off-white solid.

¹H NMR (DMSO-d₆, 80°C) δ 2.3 (s, 3H), 3.7 (m, 1H), 4.0 (m, 2H), 4.4 (m, 2H),5.8 (s, 2H), 7.55 (dd, 1H), 7.65 (d, 1H), 7.8 (d, 1 H).

LCMS (method A): retention time 3.77 minutes (M+H⁺) 304.

### Example 2: Preparation of [1-(2-Ethylamino-8-chlorobenzo[4,5]furo[3,2d]-pyrimidin-4-yl)azetidin-3-yl]N-ethyl amine

### Preparation of tert-Butyl [1-(2-amino-8-chlorobenzo[4,5]furo[3,2-d]pyrimidin-4-yl)azetidin-3-yl] N-methyl carbamate

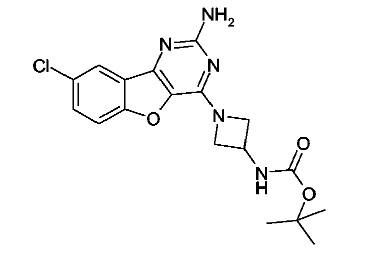

This was prepared in an analogous fashion to the preparation of *tert-*Butyl N-[1-(2-amino-8-chlorobenzo[4,5]furo[3,2-d]pyrimidin-4-yl)azetidin-3-yl] N-methyl carbamate replacing *tert-*butyl N-(azetidin-3-yl) N-methyl carbamate with *tert-*butyl azetidin-3-yl carbamate.

¹H NMR (CDCl₃) δ 1.5 (s, 9H), 4.25 (br, 2H), 4.7 (br, 3H), 5.0 (br, 2H), 5.1 (br, 1H), 7.4 (d, 1H), 7345 (dd, 1 H), 8.0 (d, 1 H).

### Preparation of tert-Butyl [1-(2-ethylamino-8-chlorobenzo[4,5]furo[3,2-d]-pyrimidin-4-yl)azetidin-3-yl] N-ethyl carbamate

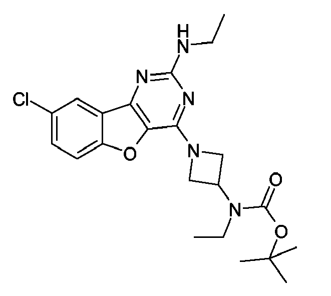

A mixture of *tert*-butyl [1-(2-amino-8-chlorobenzo[4,5]furo[3,2-d]pyrimidin-4-yl)azetidin-3-yl] N-methyl carbamate (0.05g) and sodium hydride (60% oil dispersion, 0.05g), in THF (5mL) was sonicated under an atmosphere of argon for 10 minutes. Iodoethane (1mL) was added and the mixture was sonicated for 2 hours. The resultant mixture was treated with aqueous ammonium chloride solution and extracted with ethyl acetate. The organic layer was dried (MgSO₄) and filtered and the filtrate was evaporated to dryness to give crude *tert*-butyl [1-(2-ethylamino-8-chlorobenzo[4,5]furo[3,2-d]-pyrimidin-4-yl)azetidin-3-yl] N-ethyl carbamate which was used without further purification.

### Product: [1-(2-Ethylamino-8-chlorobenzo[4,5]furo[3,2-d]-pyrimidin-4-yl)azetidin-3-yl] N-ethyl amine

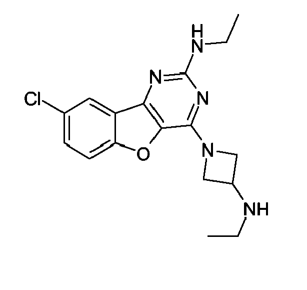

This compound was prepared starting from crude *tert*-butyl [1-(2-ethylamino-8-chlorobenzo[4,5]furo[3,2-d]-pyrimidin-4-yl)azetidin-3-yl] N-ethyl carbamate by following a similar procedure to that used for the preparation of example 1.

¹H NMR (DMSO-d₆) δ 1.0 (t, 3H), 1.1 (t, 3H), 2.55 (q, 2H), 3.3 (q, 2H), 3.75 (m, 1H), 4.0 (br, 2H), 4.45 (br, 2H), 6.6 (t, 1H), 7.6 (dd, 1H), 7.7 (d, 1H), 7.9 (d, 1H).

LCMS (method A): retention time 4.57 minutes (M+H⁺) 346.

### Example 3 Radioligand binding assay

Compounds of the invention are tested in the radioligand binding assay using human histamine H4 receptor transfected CHO K1 membranes as described above.

Compounds of the invention typically demonstrate Kᵢ values in this assay of ≤ 10 µM.

## Claims

1. A compound having the general formula (I) 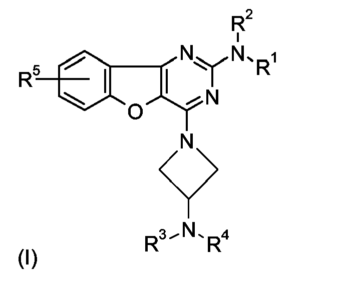 or a pharmaceutically acceptable salt, ester, prodrug or metabolite thereof, wherein
R¹ and R² are independently selected from the group consisting of H, -C(O)CH₃, -S(O)₂CH₃, CH₃, CH₂CH₃, CH₂F, CHF₂, CF₃, and cyclopropyl;
R³ and R⁴ are independently selected from the group consisting of H, CH₃, CH₂CH₃, CH₂F, CHF₂, CF₃, and cyclopropyl;
R⁵ is H, F, Cl, Br, CH₃, CN, CF₃, OH, OCH₃, or OCF₃.

2. A compound according to claim 1, wherein R¹, R² are independently selected from the group consisting of H, CH₃, and CH₂CH₃.

3. A compound according to claim 1 or 2, wherein R¹ and R² are H, R¹ and R² are CH₃ or when R¹ and R² are different one of them is H.

4. A compound according to any of claims 1 to 3, wherein R³, R⁴ are independently selected from the group consisting of H, CH₃, and CH₂CH₃.

5. A compound according to any of claims 1 to 4, wherein R³ and R⁴ are H, or when R³ and R⁴ are different one of them is H.

6. A compound according to any of claims 1 to 5, wherein R⁵ is in 8-position.

7. A compound according to any of claims 1 to 6, wherein R⁵ is Cl.

8. [1-(2-Ethylamino-8-chlorobenzo[4,5]furo[3,2-d]-pyrimidin-4-yl)azetidin-3-yl] N-ethyl amine;
N-[1-(2-Amino-8-chlorobenzo[4,5]furo[3,2-d]pyrimidin-4-yl)azetidin-3-yl] N-methyl amine.

9. A pharmaceutical composition comprising a compound of any of claims 1 to 8 in a mixture with an inert carrier.

10. A compound according to any of claims 1 to 8 for use as a medicament.

11. Use of a compound according to any of claims 1 to 8 for the manufacture of a medicament for the treatment or prophylaxes of one or more diseases or disorders associated with the modulation of histamine H4 receptor, especially with the inflammatory response mediated by histamine H4 receptor.

12. Use of a compound according to any of claims 1 to 8 for the manufacture of a medicament for the treatment or prophylaxes of inflammatory diseases.

13. Use of a compound according to any of claims 1 to 8 for the manufacture of a medicament for the treatment or prophylaxes of one or more diseases or disorders selected from the group consisting of asthma, psoriasis, rheumatoid arthritis, Crohn's disease, inflammatory bowel disease, ulcerative colitis, allergic rhinitis and other allergic diseases, atopic dermatitis and other dermatological disorders.

14. A method for treating, controlling, delaying or preventing in a mammal, preferably a human patient, in need of treatment of one or more conditions associated with the modulation of histamine H4 receptor, wherein the method comprises the administration of said patient of a pharmaceutically effective amount of a compound according to any of claims 1 to 8.

15. A process for the preparation of a compound according to any of the claims 1 to 8, comprising the steps of:
• Reacting an appropriately substituted pyrimidine of formula (VIII) with the appropriate boronic acid under conditions to form a biaryl of formula (VII). 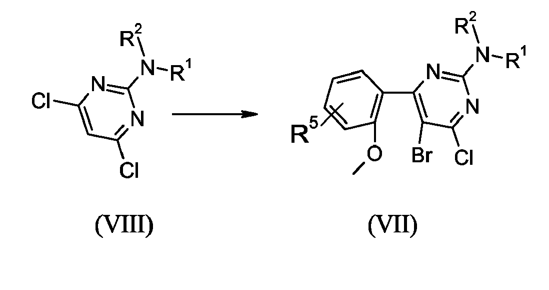
• Reacting a compound of formula (VII), with a suitable Lewis acid to form a compound of formula (VI) 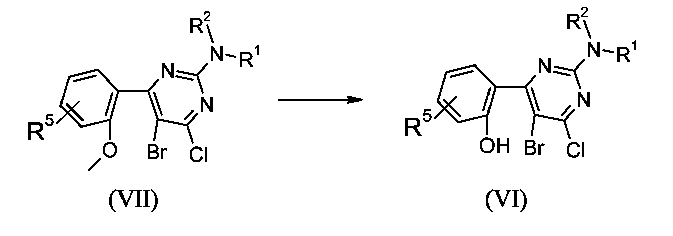
• Treating a compound of formula (VI) with a suitable catalyst, for example copper chloride to form cyclised compounds of the formula (V). 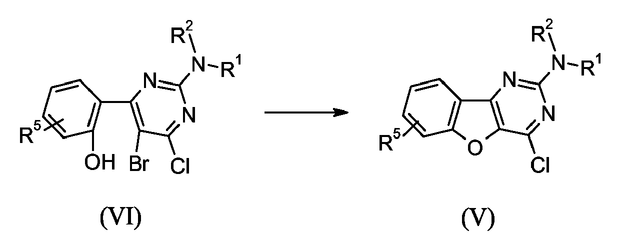
• Reacting a cyclic amine of formula (IV) with a compound of formula (V) under conditions to form a compound of general formula (I)

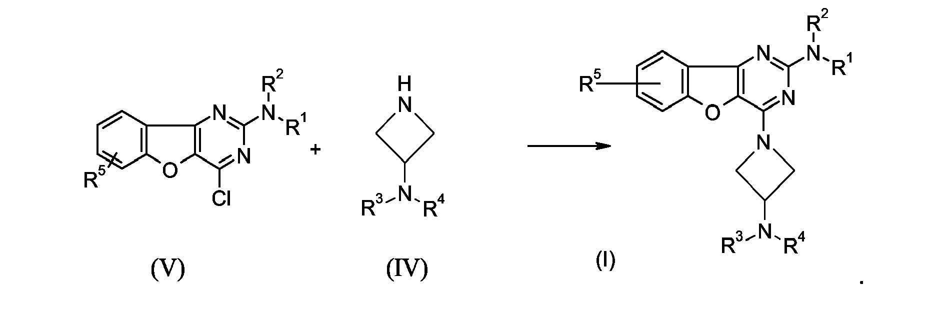

16. A process for the preparation of a medicament comprising the steps of:
a) preparing a compound according to claim 15; and
b) formulating a medicament containing said compound.
